# EUROPEAN PATENT SPECIFICATION

(11) **EP 2 276 358 B1**
(45) Date of publication and mention of the grant of the patent: **24.12.2014**
(21) Application number: 09753592.6
(22) Date of filing: 16.04.2009
(51) Int. Cl.: A23L 1/305

(54) **COMPOSITIONS FOR PROTEIN-BASED DIETARY PRODUCTS**
ERGÄNZUNGSZUSAMMENSETZUNGEN FÜR PROTEINBASIERTE DIÄTEN
Compositions de complément pour régimes à base de protéine

(30) Priority: 16.04.2008 EP 08007424
(43) Date of publication of application: 26.01.2011
(73) Proprietor: Italfarmacia S.r.l., 00155 Roma (IT)
(72) Inventor: CAVALLO, Giovanni, I-00122 Ostia (IT); CECCARELLI, Maurizio, I-00123 Roma (IT); MARCHETTI, Mario, I-00161 Roma (IT); PICCIOLI, Piero, I-00162 Roma (IT); MARCHETTI, Massimiliano, I-00167 Roma (IT)
(74) Representative: Celestino, Marco
(86) International application number: PCT/EP2009/002796
(87) International publication number: WO 2009/143937

(56) References cited:
- CN-A- 101 061 819
- US-A1- 2002 018 832
- US-B1- 6 706 697
- ANONYMOUS: "Complete Protein Powder" INTERNET ARTICLE, [Online] 20 May 2006 (2006-05-20), pages 1-2, XP002499420 Retrieved from the Internet: URL:http://web.archive.org/web/20060520081 133/http://www.buylemmon.com/product.cfm?i tem=protein> [retrieved on 2008-10-10]
- ANONYMOUS: "Organic Whey Protein Powder Supplement Meal Replacement Shake" INTERNET ARTICLE, [Online] 10 January 2008 (2008-01-10), XP002499421 Retrieved from the Internet: URL:http://web.archive.org/web/20080110062 246/http://www.frequensea-4health.com/Hood ia.html> [retrieved on 2008-10-10]
- ANONYMOUS: "Alive ! Ultra -Shake Powder" INTERNET ARTICLE, [Online] 27 August 2007 (2007-08-27), XP002499422 Retrieved from the Internet: URL:http://web.archive.org/web/20070827201 341/http://www.herbalremedies.com/whole-fo od1.html> [retrieved on 2008-10-10]
- ANONYMOUS: "Optimum Nutrition Gold Standard Whey" INTERNET ARTICLE, [Online] 2005, XP002499423 Retrieved from the Internet: URL:http://www.discount-supplements.co.uk/ products.asp?id=4191> [retrieved on 2008-10-09]
- ANONYMOUS: "Maximuscle Progain 2KG - Strawberry Dietary Supplement" INTERNET ARTICLE, [Online] 13 March 2007 (2007-03-13), XP002499424 Retrieved from the Internet: URL:http://www.amazon.co.uk/Maximuscle-Pro gain-2kg-Strawberry-Supplement/dp/B000OC1U J4> [retrieved on 2008-10-09]
- ANONYMOUS: "Cytosport Monster Milk - Nature's Ultimate Monster Muscle Formula" INTERNET ARTICLE, [Online] 13 April 2008 (2008-04-13), XP002499425 Retrieved from the Internet: URL:http://powerupz.livejournal.com/23053. html> [retrieved on 2008-10-09]
- ANONYMOUS: "Muscle Milk Chocolate - Cytosport" INTERNET ARTICLE, [Online] 9 February 2008 (2008-02-09), XP002499426 Retrieved from the Internet: URL:http://web.archive.org/web/20080209220 818/http://www.prosource.net/muscle-milk-c hocolate/29-1/1249/> [retrieved on 2008-10-09]
- ANONYMOUS: "Real Gains Universal Nutrition" INTERNET ARTICLE, [Online] 30 September 2007 (2007-09-30), XP002499427 Retrieved from the Internet: URL:http://web.archive.org/web/20070903174 436/http://shop.liveleantoday.com/product/ real-gains-universal-nutrition-muscle-buil ding-mrp-2642.cfm> [retrieved on 2008-10-13]

## Description

### Field of the invention.

The present invention relates to protein based compositions for human use dietary products, to be used in diets that are aimed at reducing body fat mass of obese or overweight patient, in particular of patients who suffer from other overweight-related diseases.

The present invention relates also to human use supplement compositions for such protein-based diets, said supplements being able to compensate for typical drawbacks of said diets.

### Background of the invention.

A feature that is often desirable in diets for treating obesity and overweight is the the effectiveness in reducing fat body mass as well as the capability to maintain, or even to contribute to increase, lean body mass. This is the case, in particular, of overweight patients that are constrained to substantial immobility by various illnesses, by whom flaccidity can be a risk.

Protein-based compositions are known for use in the cure of obesity, to cope with the risk of muscle mass loss. Proteins are composed by amino acids. The human organism is able to synthesize most of the amino acids by itself, with the exception of nine essential amino acids (Leucine, Lysine, Isoleucine, Valine, Phenilalanine, Threonine, Methionine, Histidine and Tryptophan), which must therefore be included in the diet. Essential amino acids are particularly effective to maintain lean mass if they are present in well defined proportions range.

Protein-based diets for treating obesity normally comprise a soluble dietary protein supplement containing amino acids in prefixed proportions and a protein food with low glucidic content, like meat and fish. Typically, the supplement and the food form up to 50% of the diet.A diet with low calories, based only on proteins intake, is able to reduce insulin in such extent that lipogenesis is hindered and in the same time lipolysis is stimulated, saving in the meanwhile the metabolic activity in noble tissues and assuring an equilibrated balance of nitrogen. More in detail, ingestion of essential amino acids in correct proportions stimulates the secretion of Growth Hormone, which reduces insulin. This induces the phosphorylation of the receptor of activation for the peroxisome proliferation, inhibiting the transcriptase for the adipogenesis, stimulated by the estrogens. Subsequently, the fat mobilization from the hormone dependent regions (for example, women's flanks and men's abdomen) is enhanced, without loss of lean mass. Furthermore, individuals with low serum levels of Growth Hormone tend to develop obesity, that therefore may be fought with treatments based on or able to stimulate Growth Hormone.

Most of such dietary protein-based products are not optimized to promote body fat mass reduction, and to preserve at the same time lean mass. An attempt to solve the problem has been made by adding to a protein mix such stimulating substances as caffeine and ephedrine.

Moreover, a protein based diet involves some well known drawbacks, in particular intestinal disorders like:
- constipation,
- meteorism and flatulence, caused above all by the lack of vegetable fibres, and
- bad breath, caused by the process of ketosis, when fat is broken down by the body.

As known, there is a strict association between obesity and the probability to develop type 2 or "adult onset" diabetes mellitus, the most common form of diabetes mellitus. A protein-based regimen is normally considered an useful aid in diabetes treatment. Nevertheless, according to recent investigations (Karabatas et al., P.S.E.B.M. 2000, Vol. 224: 159-165), unwanted long term side effects are possible: in particular, in a diabetic patient renal functionality may be reduced, as shown by high Glomerular Filtration Rate (GFR) values, i.e. the volume of fluid filtered by kidney glomerular capillaries per unit time); a proteins rich diet may therefore lead to deficiencies in elimination of final metabolites that result from protein catabolism and, consequently, to kidney hypertrophy, a possible cause of diabetic nephropathy.

In the light of the above, the need is felt of a protein-based dietary product which has optimal protective body lean mass preserving capacities, and which, preferably, is also useful against typical protein-based diets drawbacks. A particular interest towards such products, which are adapted to treat patients who suffer from obesity-related illnesses, such as diabetes, is also felt.

In http://www.buylemmon.com/product.cfm?item=protein, published in 2006, a dietary composition is disclosed that provides an "Organic Essential Amino Acid Blend" in addition to other components. However, not only the ratio between Lysine and Tryptophan is not disclosed, but there is no indication that to the "Organic Essential Amino Acid Blend" an additional component is added in order to correct the natural ratio between certain amino acids. This kind of dietary composition, like many others existing on the market, are based on natural protein food, and thus have ratios between amino acids as those existing in the natural protein of such food, with the drawback of not reaching ideal ratios between aminoacids.

### Summary of the invention

It is therefore a feature of the present invention to provide a human use protein-based dietary composition that is suitable for reducing body fat mass, and to prevent body lean mass reduction.

It is also a feature of the present invention to provide such a composition which is adapted to treat people that suffer from serious obesity.

It is also a feature of the present invention to provide such a human use composition which prevents constipation, which is a typical drawback of protein-based diets.

Another feature of the present invention is to provide such a human use composition which prevents meteorism and flatulence, that are other typical drawback of protein-based diets.

It also a feature of the present invention to provide such a human use composition for protein-based diets which is suitable for preventing at the same time meteorism, flatulence and constipation.

It is also a feature of the present invention to provide such a human use composition for protein-based diets which prevents bad breath, that is a further drawback of protein-based diets.

An additional feature of the present invention is to provide such a human use composition which is suitable for treating patients suffering from diabetes and other blood sugar-related illnesses.

It is a further feature of the present invention to provide such a human use supplement composition which is helpful to prevent and cure diabetic complications.

These and other features are achieved by a human use dietary product composition for treating obesity and overweight; the composition comprises:
- a protein component containing at least one protein, the protein formed by amino acid units, the amino acid units having an equivalent weight amount, the amino acid units comprising a lysine and/or tryptophan equivalent amount;
- an amino acid additional component containing an amino acid selected from the group comprised of:
   - lysine;
   - tryptophan.
The main characteristics of the composition are that the additional component contains a determined amount of lysine and/or tryptophan such that on the whole lysine and tryptophan in the composition have a weight ratio set between 2,5 and 4, and that the composition further contains a component selected from the group comprised of:
- a polysaccharide, the polysaccharide having a polymer chain, the polymer chain being composed principally of fructose units linked together by β(2→1) glycosidic bonds at the anomeric C2, the polysaccharide being characterised by a degree of polymerisation, the degree of polymerisation being the number of monomer units in the polymer chain;
- an enzyme, selected from the group comprised of:
   - α-Galactosidase;
   - Lactase;
   - a mixture of α-Galactosidase and Lactase;
   - an enzyme having a flatulence reducing action;
   - a combination thereof;
   - a combination thereof.

The composition can be either in powder form or in beverage form.

The protein component can be a milk protein, in particular a milk protein selected from the group comprised of:
- α(s1) Casein;
- α(s2) Casein;
- β-Casein;;
- k-Casein;
- β-Lactoglobulin;
- α-Lactalbumin;
- bovine serum Albumin;
- immunoglobulins;
- a combination thereof.

In particular, the weight ratio of lysine to tryptophan is set between 2,7 and 3,2.

Preferably, the polysaccharide has a terminal glucose unit.

Preferably, the composition comprises a polysaccharide of natural origin, also referred to as a natural polysaccharide. Natural polysaccharides are well known for their capacity to stimulate intestinal functionality. They acts as other soluble dietary fibre, shortening stool transit time and slightly increasing faecal bulk.

As a fermentable fibre, when metabolised by gut flora, natural polysaccharides yield short-chain fatty acids, thus producing well known favourable effects. In particular, the absorption of calcium and magnesium is enhanced, as well as the proliferation of colonic bacteria beneficial for intestinal and general health, typically Bifidobacteria and Lactobacilli (probiotic activity).

Natural polysaccharides taste from bland to slightly sweet and can advantageously be used to replace sugar, fat, and flour. Actually, the caloric content of natural polysaccharides is about 25 to 30% the food energy of sugar and other carbohydrates and ranges from one-sixth to one-ninth the caloric content of fat. Natural polysaccharides have a minimal impact on blood sugar, therefore they are suitable for diabetics.

In particular, the polysaccharide is an inulin, the inulin having a degree of polymerisation greater than 20. Energy intake is about 4 calories/g proteins, and only 1,5 calories/g inulin. Therefore, energy intake associated to the composition is even lower than the one of carbohydrates, since inulins are not absorbed an therefore are not completely hydrolized. In particular, energy intake is not more than 75 calories/dose.

In alternative, the polysaccharide is a fructooligosaccharide, also indicated with FOS

In particular, the FOS has a degree of polymerisation comprised between 3 and 10.

Fructooligosaccharides are very soluble in water because of their low degree of polymerization. They are therefore particularly well suited to produce a protein supplement in the form of a beverage.

Inulins have a greater degree of polymerization and are therefore less soluble in water than FOS, this feature making inulins better suited to produce a protein supplement in powder form. At any rate, inulins show a solubility that increases with temperature, said solubility making allowance to use inulins to obtain a protein supplement in the form of a beverage, in particular if haze or cloudiness can be tolerated.

Advantageously, the composition comprises an amount of a further essential amino acid selected from the group comprised of Leucine, Isoleucine, Valine, Phenyalanine, Threonine, Methionine, Histidine, a combination thereof, the amount adapted to substantially match a combination where amino acid units have respective weight proportions on 100% total amino acid units selected from the: Leucine 19±1%, Hisoleucine 15±1%, Valine 12±1%, Phenylalanine 10±1%, Trheonine 10±1%, Methyonine 7±1%, Histidine 7±1%.

Advantageously, the composition comprises an amino acid selected from the group comprised of:
- L-Ornithine;
- Potassium Aspartate;
- L-Taurine;
- Citrulline;
- L-Isoleucine;
- Glycine;
- L-Arginine;
- a combination thereof.

In the composition, L-Arginine may be present free or as a constituent of proteins, for instance a milk protein. As shown in recent investigations on rats (Reyes et al., Proc Soc.Exp.Biol.Med., June 1994, 206(2):157-61), L-Arginine administration can prevent kidney hypertrophy, which is a possible long-term consequence of a protein rich regimen. This effect was associated with less excretion of orotic acid in the urine. Kidney hypertrophy is to be regarded as an incipient state of diabetic nephropathy, a diabetic complication which makes it problematic to adopt protein rich diets in the case of diabetic persons.

Furthermore, the compositions according may contain Glycine and L-Taurine, free or as constituents of proteins, for instance a milk protein. Investigations [Alvarado-Vásquez et al., Life Sci. 13 Jun 2006, 79(3):225-32, Trachtman et al., Am J Physiol. Sep 1995, 269 (3 Pt 2):F429-38] have been performed on diabetic rats to establish the effectiveness of these amino acids in preventing and curing diabetic microangiopathy-related diseases. In the case of Glycine, the results gave evidence of a reduced incidence of opacity in lens and microaneurysms in the eyes, as well as a diminished expression of O-acetyl sialic acid in brain vessels compared with untreated diabetic rats. A less intense corporal weight loss in comparison with non-treated animals was also revealed. These results suggest that administration of Glycine attenuates the diabetic complications, probably due to inhibition of the non-enzymatic glycation process. In the case of L-Taurine, beneficial effects on renal functionality were observed, probably related to reduced renal oxidant injury with decreased lipid peroxidation and less accumulation of AGEs within the kidney. The compositions according to the invention can therefore provide a contribute in managing diabetic complications, which often associated to obesity.

Preferably, the composition comprises a potassium-releasing compound. In a diet composition product, potassium is important as a metabolim stimulator.

In particular, the composition contains an inulin in the range from 125 mg to 700 mg per gram proteins plus amino acids.

In particular, the composition contains a FOS in the range from 125 mg to 700 mg per gram proteins plus amino acids.

In particular, the composition contains α-Galactosidase in the range from 6 to 700 units per gram proteins plus amino acids. In particular, α-Galactosidase allows the hydrolysis of some polysaccharides (α-Galactosides) that cannot otherwise be digested in the small intestine and moves in an undigested state to the large intestine, where they are fermented, hence giving rise to gas and associated discomfort. In particular, this is the case of Raffinose, a trisaccharide which is found in such aliments as beans, cabbage, Brussels sprouts, broccoli, asparagus, other vegetables, and whole grains. Said Raffinose is hydrolysed by α-Galactosidase to D-Galactose and Sucrose, which on the contrary can be easily digested. Other polysaccharides that are advantageously treated with α-Galactosidase are Stachyose (a polysaccharide of soya bean) and Melibiosc.

In particular, the composition contains Lactase in the range from 3 to 350 units per gram proteins plus amino acids. Lactase, a β-Galactosidase, is involved in the hydrolysis of Lactose (a disaccharide, in particular a β-galactoside) into constituent Galactose and Glucose. Deficiency or lack of this enzyme is the cause of the well known Lactose intolerance. Other β-Galactosidase can be alternatively used, as they are capable of having a flatulence reducing action. The only important side effect of Inulin and FOS, as well as of other constipation reducing polysaccharides, is flatulence, for they undergo the same decomposition mechanism of other polysaccharides that cannot be digested before the large intestine, hence the utility of specific anti-flatulence enzymes in a protein supplement composition containing inulin and FOS. This is the case, for instance, of endo-inulinase. In other words, antiflatulence enzymes and anti-constipation polysaccharides have synergetic effects. Besides, compositions containing dietary components selected from both groups are to be preferred because constipation and flatulence, as protein diet associated drawbacks, usually take place together.

### Description of preferred embodiments

The composition according to the present invention will be made clearer by the following examples, that are not limitative.

With reference to tables 3 to 6B, 36 examples of supplement formulations based on a composition according to the invention are shown. All of them comprise:
- a milk protein mixture, as the protein source; a typical amino acid profile of said milk proteins is presented in Table 1. While the qualitative composition of amino acids is always defined, the percentage of each component may change (±25%), according to the process by which the proteins mixture is obtained, tipically according to the effectiveness of extraction and filtration operations.
- a combination of free amino acids, comprising
   - essential amino acids, which are added in such an amount to achieve a desired essential amino acid units combination, as shown in table 2. In particular, formulations are shown in which the ratio of Lysine to Tryptophan is equal to 3,2 or 4. In the exemplary formulations, free L-Isoelucine and Tryptophan are added to protein mixture to match with the above mentioned preferred amino acid units combination.
   - other amino acids, in particular L-Ornithine, Aspartic Acid in the form of Potassium Aspartate, L-Taurine, Tryptophan, L-Isoleucine, Citrulline.
- Table 3A presents three powder proteins - amino acids -Inulin (PAIP) formulations, which only differ from one another in the amount of Inulin, in a common protein-amino acids basic formulation; Inulin amount ranges from about 200 mg to 665 mg per gram milk proteins plus amino acids; in all the formulations the Lysine-to-Tryptophan weight ratio is 4;
- Table 3B presents three further PAIP formulations, which also differ from one another in the amount of Inulin, ranging from about 200 mg to 665 mg per gram milk proteins plus amino acids; in this case the Lysine-to-Tryptophan weight ratio is 3,2;
- Table 4A presents six powder proteins - amino acids -enzymes (PAEP) formulations, which only differ from one another in the amount of enzymes, a 2:1 units combination of α-Galactosidase and Lactase, in a common protein-amino acids basic formulation; Enzymes range from 10 to 1000 units per gram milk proteins plus amino acids; in all the formulations the Lysine-to-Tryptophan weight ratio is 4;
- Table 4B presents six further PAEP formulations, which also differ from one another in the amount of the 2:1 combinations of Enzymes, ranging from from 10 to 1000 units per gram milk proteins plus amino acids; in this case the Lysine-to-Tryptophan weight ratio is 3,2;
- Table 5A presents three beverage proteins - amino acids -Inulin (PAFB) formulations, which only differ from one another in the amount of FOS, in a common protein-amino acids basic formulation; Inulin amount ranges from about 200 mg to 665 mg per gram milk proteins plus amino acids; in all the formulations the Lysine-to-Tryptophan weight ratio is 4;
- Table 5B presents three further PAFB formulations, which also differ from one another in the amount of FOS, ranging from about 200 mg to 665 mg per gram milk proteins plus amino acids; in this case the Lysine-to-Tryptophan weight ratio is 3,2;
- Table 6A presents six powder proteins - amino acids -enzymes (PAEB) formulations, which only differ from one another in the amount of enzymes, a 2:1 units combination of α-Galactosidase and Lactase, in a common protein-amino acids basic formulation; Enzymes range from 10 to 1000 units per gram milk proteins plus amino acids; in all the formulations the Lysine-to-Tryptophan weight ratio is 4;
- Table 6B presents six further PAEB formulations, which also differ from one another in the amount of the 2:1 combinations of Enzymes, ranging from 10 to 1000 units per gram milk proteins plus amino acids; in this case the Lysine-to-Tryptophan weight ratio is 3,2.

To achieve an enhanced slimming effect, the powder composition according to the invention is administrated according to the following steps:
- starting treatment (3 weeks)
   - for women: 1,2 g protein and amino acid /kg ideal body weight/ day, corresponding to an average of hree 15 g composition packets,
   - for men: 1,5 g protein and amino acid /kg ideal body weight /day, corresponding to an average of three 15 g protein/aminoacid matter packets

Patients should every day drink at least 2 litres of water, corresponding to four 15 g protein/amino acid substance.
maintaining treatment (2 weeks), to be made after a break of two weeks, during which the patients follow a low caloric diet. One 15 g protein/amino acid substance wil be taken a day, preferably in the morning.

Another possibility is the administration of the composition via a nasogastric tube. 15 g of powder composition are dissolved in about 2 litres of water, and the resulting solution is stored into an infusion bag. A continuous administration is then provided through a nasogastric tube, preferably by using a peristaltic pump. The administration is made according to the following steps:
- starting treatment (3 weeks)
   - for women: 1 g protein and amino acid /kg ideal body weight/ day;
   - for men: 1,2 g protein and amino acid /kg ideal body weight /day;
maintaining treatment (2 weeks), to be made after a break of two weeks, during which the patients follow a low caloric diet. One 15 g protein/amino acid substance packet will be taken a day, preferably in the morning.

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

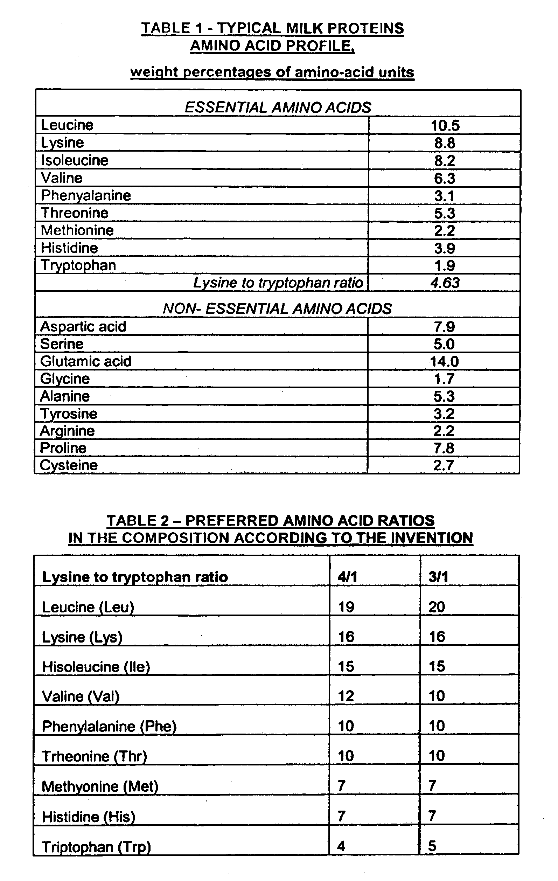

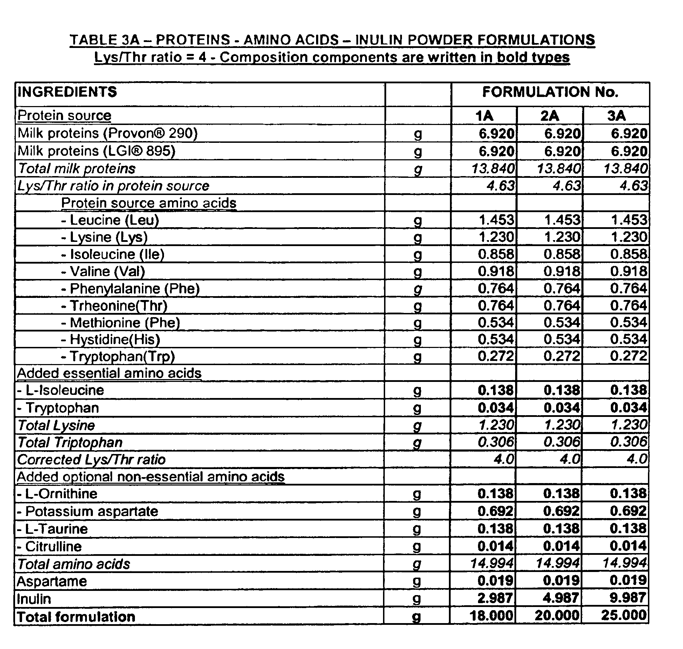

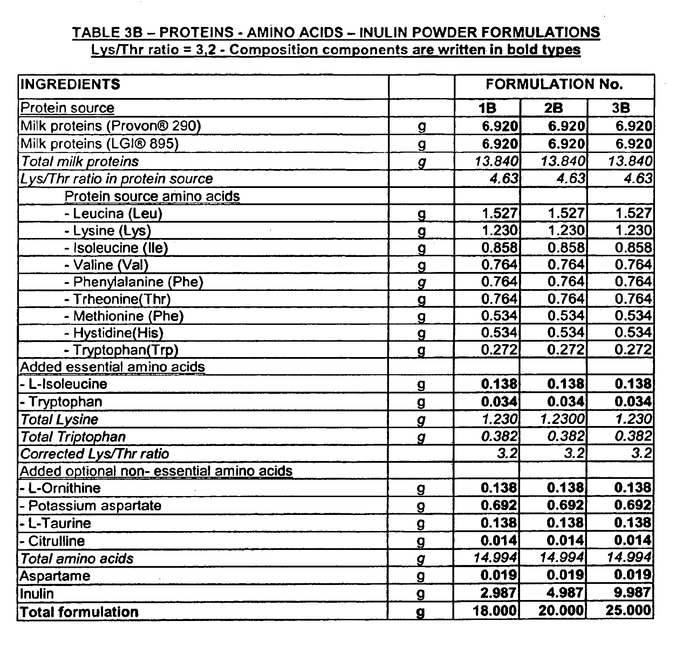

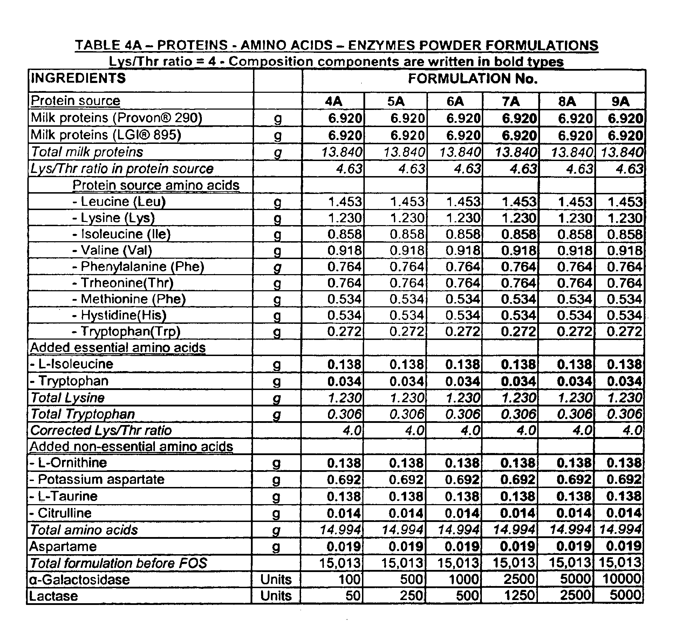

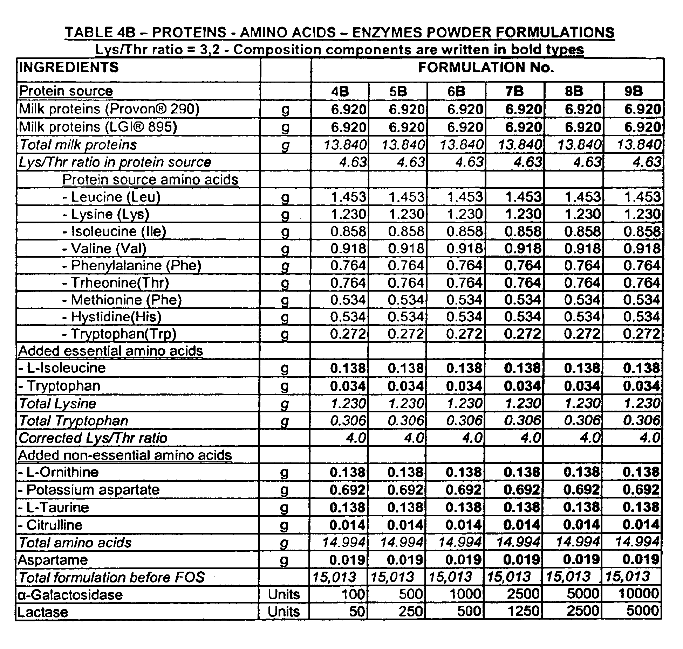

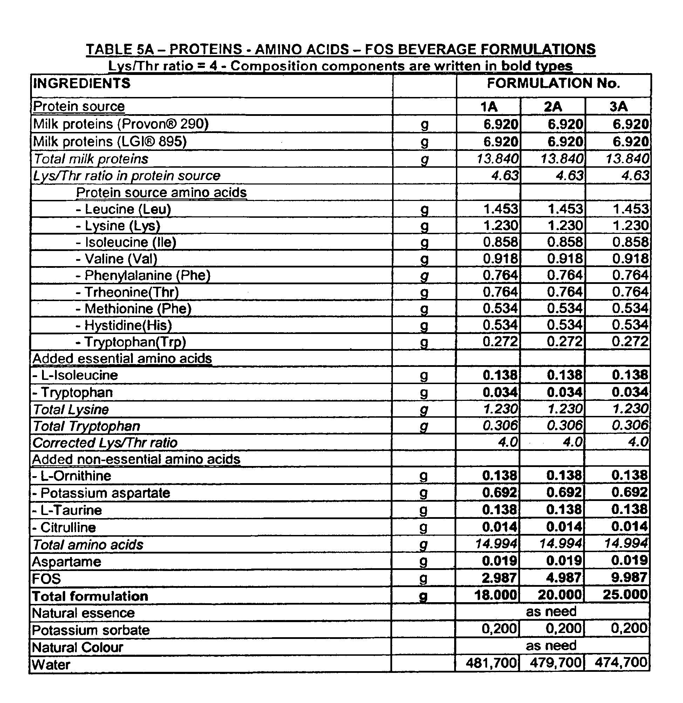

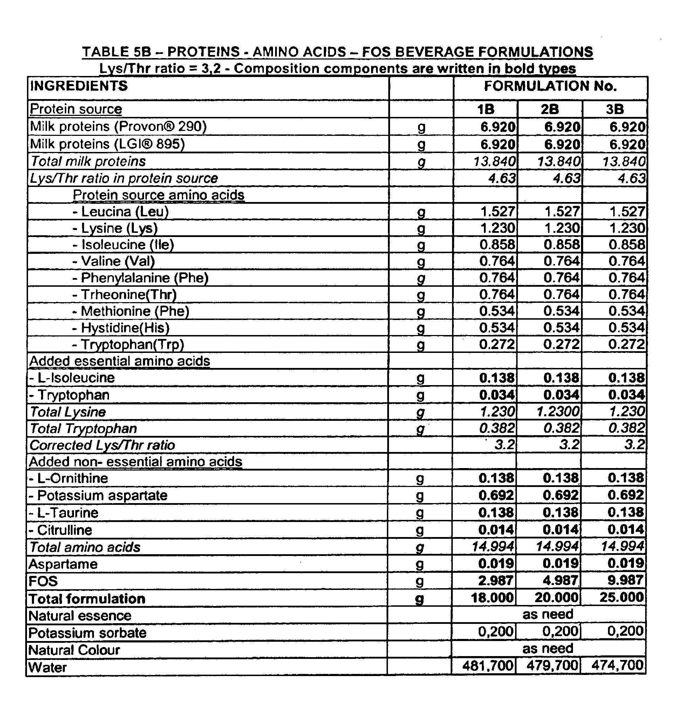

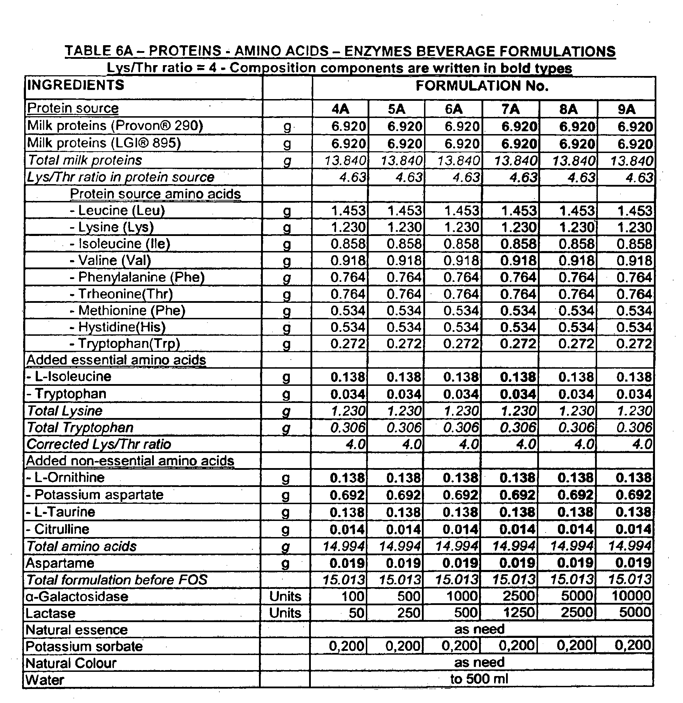

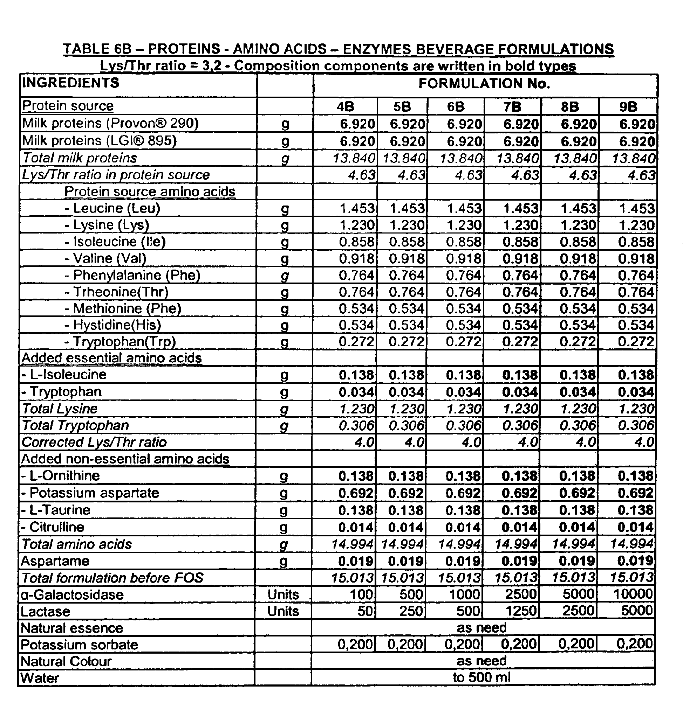

## Claims

1. A human use dietary product composition for treating obesity and overweight, said composition comprising:
- a protein component containing at least one protein, said protein formed by amino acid units;
- an amino acid additional component containing an amino acid selected from the group comprised of:
- lysine;
- tryptophan;
- a combination thereof,
**characterised in that** said additional component contains a determined amount of said lysine and/or tryptophan such that by adding said additional component to said protein component said composition provides a desired essential amino acid units combination where on the whole said lysine and tryptophan in said composition have a weight ratio set between 2,5 and **4,and in that** said composition further contains a component selected from the group comprised of:
- a polysaccharide, said polysaccharide having a polymer chain, said polymer chain being composed principally of fructose units linked together by β(2→1) glycosidic bonds at the anomeric C2, said polysaccharide being **characterised by** a degree of polymerisation, said degree of polymerisation being the number of monomer units in said polymer chain;
- an enzyme, selected from the group comprised of:
- α-Galactosidase;
- Lactase;
- a mixture of α-Galactosidase and Lactase;
- an enzyme having a flatulence reducing action;
- a combination thereof;
- a combination thereof.

2. A composition according to claim 1, wherein said weight ratio is set between 2,7 and 3,2.

3. A composition according to claim 1, wherein said polysaccharide has a terminal glucose unit.

4. A composition according to claim 1, wherein said polysaccharide is an inulin, said inulin having a degree of polymerisation greater than 20.

5. A composition according to claim 1, wherein said polysaccharide is a fructooligosaccharide, also indicated with FOS

6. A composition according to claim 5, wherein said FOS has a degree of polymerisation comprised between 3 and 10.

7. A composition according to claim 1, comprising furthermore an amount of a further essential amino acid selected from the group comprised of Leucine, Isoleucine, Valine, Phenyalanine, Threonine, Methionine, Histidine, a combination thereof, said amount adapted to substantially match a combination where amino acid units have respective weight proportions on 100% total amino acid units selected from the: Leucine 19±1%, Hisoleucine 15±1%, Valine 12±1%, Phenylalanine 10±1%, Trheonine 10±1%, Methyonine 7±1%, Histidine 7±1%.

8. A composition according to claim 1, comprising furthermore an amino acid selected from the group comprised of:
- L-Ornithine;
- Potassium Aspartate;
- L-Taurine;
- Citrulline;
- L-Isoleucine;
- Glycine;
- L-Arginine;
- a combination thereof.

9. A composition according to claim 1, comprising furthermore a potassium-releasing compound.

10. A composition according to claim 1, wherein said composition contains an inulin in the range from 125 mg to 700 mg per gram proteins plus amino acids.

11. A composition according to claim 1, wherein said composition contains a FOS in the range from 125 mg to 700 mg per gram proteins plus amino acids.

12. A composition according to claim 1, wherein said composition contains α-Galactosidase in the range from 6 to 700 units per gram proteins plus amino acids.

13. A composition according to claim 1, wherein said composition contains Lactase in the range from 3 to 350 units per gram proteins plus amino acids.

## Patentansprüche

1. Diätetische Zusammensetzung zur Behandlung von Fettleibigkeit und Übergewicht für den menschlichen Gebrauch, wobei die Zusammensetzung umfasst:
- eine Proteinkomponente, die mindestens ein Protein enthält, wobei das Protein aus Aminsäureeinheiten besteht,
- eine zusätzliche Aminosäurekomponente, die eine Aminosäure enthält, welche aus der Gruppe bestehend aus:
- Lysin,
- Tryptophan,
- einer Kombination hiervon
ausgewählt ist,
**dadurch gekennzeichnet, dass** die zusätzliche Komponente eine vorbestimmte Menge des Lysins und/oder des Tryptophans enthält, so dass die Zusammensetzung durch Zugabe der zusätzlichen Komponente zu der Proteinkomponente eine gewünschte Kombination an essentiellen Aminosäureeinheiten bereitstellt, wobei das Lysin und das Tryptophan in der Zusammensetzung insgesamt in einem Gewichtsverhältnis vorliegen, das auf einen Wert zwischen 2.5 und 4 eingestellt ist,
und dass die Zusammensetzung weiterhin eine Komponente enthält, die aus der Gruppe bestehend aus:
- einem Polysaccharid, wobei das Polysaccharid eine Polymerkette aufweist und die Polymerkette hauptsächlich aus Fructoseeinheiten besteht, die über β(2-1)-glykosidische Bindungen am anomeren C2 miteinander verknüpft sind, und wobei das Polysaccharid durch einen Polymerisationsgrad gekennzeichnet ist und der Polymerisationsgrad die Anzahl an Monomereinheiten in der Polymerkette bezeichnet,
- einem Enzym ausgewählt aus der Gruppe bestehend aus:
- α-Galactosidase,
- Lactase,
- einem Gemisch von α-Galactosidase und Lactase,
- einem Enzym mit entblähender Wirkung,
- einer Kombination hiervon,
- einer Kombination hiervon
ausgewählt ist.

2. Zusammensetzung nach Anspruch 1, wobei das Gewichtsverhältnis auf einen Wert zwischen 2.7 und 3.2 eingestellt ist.

3. Zusammensetzung nach Anspruch 1, wobei das Polysaccharid eine terminale Glucoseeinheit aufweist.

4. Zusammensetzung nach Anspruch 1, wobei das Polysaccharid ein Inulin ist und das Inulin einen Polymerisationsgrad von mehr als 20 aufweist.

5. Zusammensetzung nach Anspruch 1, wobei das Polysaccharid ein Fructooligosaccharid, auch als FOS bezeichnet, ist.

6. Zusammensetzung nach Anspruch 5, wobei das FOS einen Polymerisationsgrad von zwischen 3 und 10 aufweist.

7. Zusammensetzung nach Anspruch 1, weiterhin umfassend eine bestimmte Menge einer weiteren essentiellen Aminosäure ausgewählt aus der Gruppe bestehend aus Leucin, Isoleucin, Valin, Phenylalanin, Threonin, Methionin, Histidin, einer Kombination hiervon, wobei die Menge derart angepasst ist, dass sie im Wesentlichen mit einer Kombination übereinstimmt, in der die Aminosäureeinheiten, bezogen auf eine Gesamtheit von 100% Aminosäureeinheiten, jeweilige Gewichtsanteile ausgewählt aus: Leucin 19±1%, Isoleucin 15±1%, Valin 12±1%, Phenylalanin 10±1%, Threonin 10±1%, Methionin 7±1%, Histidin 7±1% besitzen.

8. Zusammensetzung nach Anspruch 1, weiterhin umfassend eine Aminosäure ausgewählt aus der Gruppe bestehend aus:
- L-Ornithin,
- Kaliumaspartat,
- L-Taurin,
- Citrullin,
- L-Isoleucin,
- Glycin,
- L-Arginin,
- einer Kombination hiervon.

9. Zusammensetzung nach Anspruch 1, weiterhin umfassend eine Kalium-freisetzende Verbindung.

10. Zusammensetzung nach Anspruch 1, wobei die Zusammensetzung im Bereich von 125 mg bis 700 mg eines Inulins pro Gramm Proteine plus Aminosäuren enthält.

11. Zusammensetzung nach Anspruch 1, wobei die Zusammensetzung im Bereich von 125 mg bis 700 mg eines FOS pro Gramm Proteine plus Aminosäuren enthält.

12. Zusammensetzung nach Anspruch 1, wobei die Zusammensetzung im Bereich von 6 bis 700 Einheiten an α-Galactosidase pro Gramm Proteine plus Aminosäuren enthält.

13. Zusammensetzung nach Anspruch 1, wobei die Zusammensetzung im Bereich von 3 bis 350 Einheiten an Lactase pro Gramm Proteine plus Aminosäuren enthält.

## Revendications

1. Composition de produit diététique à usage humain pour le traitement de l'obésité et du surpoids, ladite composition comprenant :
- un composant protéine contenant au moins une protéine, ladite protéine étant formée par des unités d'acides aminés ;
- un composant acide aminé supplémentaire contenant un acide aminé choisi dans le groupe constitué par ;
- la lysine ;
- le tryptophane ;
- une combinaison de ceux-ci,
**caractérisée en ce que** ledit composant supplémentaire contient une quantité déterminée de ladite lysine et/ou dudit tryptophane telle que l'ajout dudit composant supplémentaire audit composant protéine permette à ladite composition de fournir une combinaison souhaitée d'unités d'acides aminés essentiels, ladite lysine et ledit tryptophane présentant dans l'ensemble dans ladite composition un rapport en poids compris entre 2,5 et 4, et **en ce que** ladite composition contient en outre un composant choisi dans le groupe constitué par :
- un polysaccharide, ledit polysaccharide comprenant une chaîne polymère, ladite chaîne polymère étant composée principalement d'unités fructose reliées ensemble par des liaisons β(2→1) glycosidiques au niveau du C3 anomérique, ledit polysaccharide étant **caractérisé par** un degré de polymérisation, ledit degré de polymérisation étant le nombre d'unités monomères dans ladite chaîne polymère ;
- une enzyme, choisie dans le groupe constitué par :
- l'α-galactosidase ;
- la lactase ;
- un mélange d'α-galactosidase et de lactase ;
- une enzyme ayant une action de réduction des flatulences ;
- une combinaison de ceux-ci ;
- une combinaison de ceux-ci.

2. Composition selon la revendication 1, dans laquelle ledit rapport en poids est compris entre 2,7 et 3,2.

3. Composition selon la revendication 1, dans laquelle ledit polysaccharide comprend une unité glucose terminale.

4. Composition selon la revendication 1, dans laquelle ledit polysaccharide est une inuline, ladite inuline ayant un degré de polymérisation supérieur à 20.

5. Composition selon la revendication 1, dans laquelle ledit polysaccharide est un fructooligosaccharide, également nommé FOS.

6. Composition selon la revendication 5, dans laquelle ledit FOS a un degré de polymérisation compris entre 3 et 10.

7. Composition selon la revendication 1, comprenant en outre une quantité d'un acide aminé essentiel supplémentaire choisi dans le groupe constitué par la leucine, l'isoleucine, la valine, la phénylalanine, la thréonine, la méthionine, l'histidine, une combinaison de celles-ci, ladite quantité étant conçue pour correspondre essentiellement à une combinaison selon laquelle les unités d'acides aminés ont des proportions en poids respectives par rapport à 100 % au total d'unités d'acides aminés choisies parmi : 19 ± 1 % de leucine, 15 ± 1 % d'isoleucine, 12 ± 1 % de valine, 10 ± 1 % de phénylalanine, 10 ± 1 % de thréonine, 7 ± 1 % de méthionine, 7 ± 1 % d'histidine.

8. Composition selon la revendication 1, comprenant en outre un acide aminé choisi dans le groupe constitué par :
- la L-ornithine ;
- l'aspartate de potassium ;
- la L-taurine ;
- la citrulline ;
- la L-isoleucine ;
- la glycine ;
- la L-arginine ;
- une combinaison de ceux-ci.

9. Composition selon la revendication 1, comprenant en outre un composé libérant du potassium.

10. Composition selon la revendication 1, dans laquelle ladite composition contient une inuline dans la plage allant de 125 mg à 700 mg par gramme de protéines plus acides aminés.

11. Composition selon la revendication 1, dans laquelle ladite composition contient un FOS dans la plage allant de 125 mg à 700 mg par gramme de protéines plus acides aminés.

12. Composition selon la revendication 1, dans laquelle ladite composition contient une α-galactosidase dans la plage allant de 6 à 700 unités par gramme de protéines plus acides aminés.

13. Composition selon la revendication 1, dans laquelle ladite composition contient une lactase dans la plage allant de 3 à 350 unités par gramme de protéines plus acides aminés.
